# EUROPEAN PATENT APPLICATION

(11) **EP 4 749 288 A1**
(43) Date of publication of application: **27.05.2026**
(21) Application number: 24215255.1
(22) Date of filing: 25.11.2024
(51) Int. Cl.: G01N 33/58, G01N 33/543, G01N 33/569

(54) **COMPOSITIONS FOR DETECTING, CHARACTERIZING, AND REMOVING A BIOFILM**

(71) Applicant: COSA Group GmbH, 80539 München (DE)
(72) Inventor: POTRECK, Janine-Melanie, 80539 München (DE)
(74) Representative: Maiwald GmbH

(57) **Abstract**

The present disclosure is directed to a composition for detecting and/or localizing and/ or characterization of a biofilm and/or for removing a biofilm, methods for detecting, characterizing, and/or removing a biofilm, and the use of a composition for detecting, characterizing, and/or removing a biofilm, such as in pharma and food industry, water treatment and supply, oil and gas industry, medical devices and implants, environmental technology, dental industry, biotechnology and research laboratories, and food packaging.

## Description

### Technical field

The present disclosure lies in the field of compositions for detecting a biofilm, characterizing a biofilm, and/or for removing a biofilm, as well as methods for detecting, characterizing, and/or removing a biofilm, and the use of a composition for detecting, characterizing, and/or removing a biofilm, such as in pharma and food industry, water treatment and supply, oil and gas industry, medical devices and implants, environmental technology, dental industry, biotechnology and research laboratories, and food packaging.

### Background

A biofilm is a structured community of microorganisms (such as bacteria, fungi, and/or algae) that adhere to a surface and are embedded within a self-produced matrix of extracellular polymeric substances (EPS). This matrix, which is often composed of polysaccharides, proteins, and nucleic acids, helps the microorganisms stick together and attach to surfaces.

This structure makes them particularly resistant to external influences, such as antibiotic treatments, disinfectants, and mechanical stress. Therefore, the formation of biofilms on a surface is a major challenge, especially in industrial cleaning processes and medical industry.

In the medical industry, biofilms often - especially when a biofilm is formed on the surface of production equipment, an implant, catheter, or other medical device - cause major risks of infections for a patient. In particular, infections caused by a biofilm formed on the surface of an implant are especially difficult to treat due to their specific structure and protection mechanisms against antibiotics. In the food industry, biofilms on production equipment, food packaging, or the food itself cause contamination risks that endanger both product safety and consumer health. In the water and wastewater industry, biofilms can form in pipes or filter systems and clog or damage them, leading to high maintenance costs alongside potential health risks.

The detection, classification, and removal of biofilms from a surface represents a particularly important task in industrial and medical cleaning processes, as well as a major challenge.

Detection of a biofilm may often be highly complicated because biofilms are often invisible and distributed over a surface in an irregular pattern. Therefore, efficient detection methods that are both sensitive and suitable for detecting a biofilm on large surfaces are urgently needed.

Also, the classification of a biofilm, which may determine the methods of removing it, is highly tedious or even impossible without the use of special knowledge and microbiological analysis.

Even if a biofilm could be detected, it is still particularly difficult to remove. In particular, removing the biofilm must be effective without damaging the treated surface or creating new risks, such as the spread of pathogenic microorganisms.

Previous options for detecting a biofilm on the surface, such as fluorescence microscopy, may be precise but only suitable for small samples and require complex sample preparation, limiting their scalability to larger areas. Raman spectroscopy provides detailed chemical information but is expensive and time consuming, making it impractical for real-time applications.

Other imaging technologies such as hyperspectral cameras can cover large areas, but are costly and sensitive to surface roughness or contaminations, which can affect accuracy. Sensor-based approaches such as electrochemical detectors or biosensitive surfaces enable real-time measurements, but are often susceptible to environmental conditions such as temperature and humidity. Molecular methods such as DNA sequencing provide precise information about biofilm composition, but require long processing times, expensive equipment and specialized personnel.

In addition, some methods, such as chemical staining, have the disadvantage that they change the biofilm structure and therefore make precise analysis more difficult. These specific limitations highlight the need to further develop detection methods to ensure broader applicability and greater efficiency.

These challenges require integrated approaches that are coordinated which each other in order to combat biofilms efficiently.

### Summary of the invention

In view of the above, the present invention is directed to overcoming the needs for simple and convenient methods of detecting and/or localizing a biofilm, characterizing such biofilm, and/or removing such biofilm. In particular, the methods and systems provided herein are not only easy to use without specific knowledge of microbiology and without the use of complicated and expensive equipment, but are also robust and reliable.

The inventor of the present disclosure has surprisingly found that compositions as disclosed herein offer huge advantages in detecting and/or localizing and/or characterization and/or removing a biofilm with high accuracy and low technological requirements. Thereby, the compositions of the present disclosure employ standard techniques for incorporating labels for detecting a biofilm into a substance portion and at the same time can be adapted to selectively bind to a biofilm in a specific given stage of its evolution for characterizing a biofilm. Thus, the composition according to the present invention is not only adapted to detect a biofilm, but at the same time may characterize the biofilm. Additionally, the present invention also allows for the safe and efficient removal of biofilms. Therefore, the present invention addresses the above-mentioned needs.

The present invention is defined by the appended claims.

### Detailed description of the invention

In the following, the present invention will be explained with reference to preferred embodiments. However, the present invention is only defined by the appended claims.

In essence, the present invention relates to the following aspects: In a first aspect, the present invention relates to a composition for detecting and/or localizing a biofilm as defined in the appended claims. In a second aspect, the present invention relates to a method for detecting and/or localizing a biofilm as defined in the appended claims. In a third aspect, the present invention relates to a use of the composition according to the first aspect for detecting and/or localizing a biofilm as defined in the appended claims.

In a fourth aspect, the present invention relates to a composition for characterizing a biofilm. In a fifth aspect, the present invention relates to a method for characterizing a biofilm. In a sixths aspect, the present invention relates to a use of the composition according to the fourth aspect for characterizing a biofilm.

In a seventh aspect, the present invention relates to a composition for removing a biofilm. In an eighth aspect, the present invention relates to a method of removing a biofilm. In a ninth aspect, the present invention relates to a use of the composition according to the seventh aspect for removing a biofilm.

### Detecting a biofilm

In a first aspect, the present invention relates to composition for detecting and/or localizing a biofilm, preferably a biofilm on a surface. In that, the composition comprises a plurality of first labeled substance portions (K_{L}1), wherein each of the first labeled substance portions (K_{L}1) comprises a first dye as defined herein, and wherein the first labeled substance portions (K_{L}1) have a mean particle diameter in the range of from about 1 nm to about 40 µm, preferably in the range of from about 1 nm to about 20 µm.

According to another preferred embodiment, the first labeled substance portions (K_{L}1) have a mean particle diameter of at least about 10 nm, preferably at least about 50 nm, more preferably at least about 100 nm, yet more preferably about 200 nm, most preferably at least about 500 nm.

The inventor of the present disclosure has surprisingly found, that adapting the mean particle diameter of the first labeled substance portions (K_{L}1) in the range of from about ~1 nm to about 40 µm, preferably of from about ~1 nm to about 20 µm, allows the labeled substance portions (K_{L}1) to infiltrate or diffuse into the pores of a biofilm thus allowing for a detection of the first dye comprised in the first labeled substance portions (K_{L}1) within the biofilm. Therefore, the composition according to the first aspect of the present invention allows for a simple and convenient detection and/or localization of a biofilm, such as a biofilm on a surface.

According to a preferred embodiment, each of the first labeled substance portions (K_{L}1) is provided with at least one first functional group (F_{L}1) as defined herein and at least one first linker (L_{L}1) as defined herein, wherein the at least one first linker (L_{L}1) is adapted to bind the at least one first functional group (F_{L}1) to the at least one first labeled substance portion (K_{L}1).

According to a preferred embodiment of the present invention, the composition according to the first aspect further comprises a plurality of second labeled substance portions (K_{L}2), wherein each of the second labeled substance portions (K_{L}2) comprises a second dye as defined herein, and wherein the second labeled substance portions (K_{L}2) have a mean particle diameter in the range of from about 3 µm to about 8 µm. Preferably, the mean particle diameter of the second labeled substance portions (K_{L}2) is different from the mean particle diameter of the first labeled substance portions (K_{L}1). It is preferable that the second labeled substance portions (K_{L}2) have a mean particle diameter in the range of from about 3 µm to about 8 µm, and the first labeled substance portions (K_{L}1) have a mean particle diameter in the range of from about 1 µm to about 4 µm, or vice versa.

In other words, the composition according to the first aspect of the present invention may comprise two different populations of substance portions, i.e. may have a bimodal particle size distribution. The inventor of the present invention has surprisingly found that providing such composition having a bimodal particle size distribution allows for determining the stage of biofilm development, such as the age of a biofilm, and detecting the biofilm at the same time. It is understood that the pore size of a biofilm may change during its evolution and development. Therefore, the pore size of the biofilm may be determined according to the diameter of the substance portions that infiltrate into the pores of said biofilm and hence may be detected.

According to a preferred embodiment, each of the second labeled substance portions (K_{L}2) is provided with at least one second functional group (F_{L}2) as defined herein and at least one second linker (L_{L}2) as defined herein, wherein the at least one second linker (L_{L}2) is adapted to bind the at least one second functional group (F_{L}2) to the at least one second labeled substance portion (K_{L}2).

According to a preferred embodiment, each of the labeled substance portions as defined herein is provided with one type of functional group as defined herein, such as -OH, -COOH, -NH₂, metal ion, lipid, or the like, preferably with one type of functional group as defined in Table 2. It is understood that each of the labeled substance portions may comprise one or more of said functional group.

According to another preferred embodiment, each of the labeled substance portions as defined herein is provided with one or more types of functional groups.

According to another preferred embodiment of the present invention, the composition according to the first aspect further comprises a plurality of third labeled substance portions (K_{L}3), wherein each of the third labeled substance portions (K_{L}3) comprises a third dye as defined herein and wherein the third labeled substance portion (K_{L}3) have a mean particle diameter are in the range of from about 6 µm to about 10 µm. Preferably, the mean particle diameter of the third labeled substance portions (K_{L}3) is different from the mean particle diameter of the first labeled substance portions (K_{L}1) and from the mean particle diameter of the second labeled substance portions (K_{L}2). It is preferable that the third labeled substance portions (K_{L}3) have a mean particle diameter in the range of from about 6 µm to about 10 µm, and the first labeled substance portions (K_{L}1) have a mean particle diameter in the range of from about 1 µm to about 4 µm, or *vice versa.* More preferably, the third labeled substance portions (K_{L}3) have a mean particle diameter in the range of from about 6 µm to about 10 µm, the second labeled substance portions (K_{L}2) have a mean particle diameter in the range of from about 3 µm to about 8 µm, and the first labeled substance portions (K_{L}1) have a mean particle diameter in the range of from about 1 µm to about 4 µm. In other words, the composition according to the first aspect of the present invention may comprise three populations of substance portions, i.e. has a trimodal particle size distribution.

According to a preferred embodiment, each of the third labeled substance portions (K_{L}3) is provided with at least one third functional group (F_{L}3) as defined herein and at least one third linker (L_{L}3) as defined herein, wherein the at least one third linker (L_{L}3) is adapted to bind the at least one third functional group (F_{L}3) to the at least one third labeled substance portion (K_{L}3).

According to another preferred embodiment, the composition may contain one or more further populations of labeled substance portions (K_{L}n). In that, it is understood that each of the one or more further populations of labeled substance portions (K_{L}n) comprises a dye as defined herein, and has a mean particle diameter that is different from the first, second, and third labeled substance portions.

According to another preferred embodiment, the first labeled substance portions (K₁L), the second labeled substance portions (K_{L}2) (if present), the third labeled substance portions (K_{L}3) (if present), and one or more further labeled substance portions (K_{L}n) (if present) each independently of one another have a mean particle diameter in the range of from about 1 nm to about 20 µm. According to another preferred embodiment, the first labeled substance portions (K₁L), the second labeled substance portions (K_{L}2) (if present), the third labeled substance portions (K_{L}3) (if present), and the one or more further labeled substance portions (K_{L}n) (if present) each independently of one another have a mean particle diameter in the range of from about 1 µm to about 4 µm, or in the range of from about 3 µm to about 8 µm, or in the range of from about 6 µm to about 10 µm.

The inventor of the present disclosure has surprisingly found that providing a composition comprising multiple, such as two, three, four, or more populations of labeled substance portions allows for characterizing the biofilm and detecting the biofilm at the same time without the need of microbiological analysis methods. It is understood that the pore size of a biofilm changes depending on its stage of evolution. In the early stages of biofilm growth and initial formation and/or attachment to a surface, said biofilm has rather large pores, i.e. the mean pore size of the biofilm in early stages of biofilm growth is about 10 µm. During maturation and growth of the biofilm, the pore size decreases. In the early stage of biofilm growth, the pore size of said biofilm may be about 8 µm. During further growth the pore size of said biofilm may further decrease to about 3 µm, whereas a fully developed biofilm has a mean pore size of about 1 µm. By providing a composition comprising multiple populations of labeled substance portions, it has been surprisingly found that substance portions having a specific mean particle diameter only bind to biofilm having a specific pore size. By detecting the respective dye as defined herein contained in the labeled substance portion binding to the biofilm, the states of biofilm development may be easily determined and the biofilm is characterized. In other words, the stage of evolution of a biofilm can be determined according to a color-code.

According to another preferred embodiment, each of the dyes as defined herein, i.e. first, second, and third dye as defined herein, is independently a fluorescent dye or a non-fluorescent dye, with the provision that the first dye, the second dye (if present), and the third dye (if present) are different from each other. In other words, it is understood that each of the types of labeled substance portions comprised in a composition according to the first aspect of the present invention comprises a different dye as defined herein. The dyes must be selected so that the colours do not overlap in their spectrum and/or fluorescence is not extinguished.

According to a further preferred embodiment, the composition according to the first aspect of the present invention further comprises a cleaning composition according to the seventh aspect of the present disclosure. According to a further preferred embodiment, the composition according to the first aspect of the present invention further comprises a composition for characterizing a biofilm according to the fourth aspect of the present disclosure.

According to another preferred embodiment of the present disclosure, each of the first labeled substance portions (K_{L}1) further comprises a cleaning agent as defined herein. According to another preferred embodiment of the present disclosure, each of the second labeled substance portions (K_{L}2) further comprises a cleaning agent as defined herein. According to another preferred embodiment of the present disclosure, each of the third labeled substance portions (K_{L}3) further comprises a cleaning agent as defined herein. According to another preferred embodiment of the present disclosure, each of the further labeled substance portions (K_{L}n) further comprises a cleaning agent as defined herein.

If any of the first labeled substance portions (K_{L}1), of the second labeled substance portions (K_{L}2), third labeled substance portions (K_{L}3), and/or further labeled substance portions (K_{L}n) further comprises a cleaning agent as defined herein, each of said labeled substance portions can be activated, preferably can be activated by at least one of a change in pressure, a change in pH, UV radiation, a change in osmotic pressure, a change in temperature, a change in light intensity, and/or by contact to a biofilm.

According to another preferred embodiment, each of the first labeled substance portions (K_{L}1), and/or each of the second labeled substance portions (K_{L}2) (if present), and/or each of the third labeled substance portions (K_{L}3) (if present), and/or each of the further labeled substance portions (K_{L}n) (if present) independently of each other has a hydrophilic surface, or a positively charged surface, or a negatively charged surface, or a lipophilic surface.

According to a second aspect, the present invention relates to a method for detecting and/or localizing a biofilm, preferably a method for detecting and/or localizing a biofilm on a surface, the method comprising the steps of a) contacting a composition according to the first aspect of the present invention with a biofilm to provide a labeled biofilm; b) washing said labeled biofilm to provide a washed biofilm; and c) detecting the composition on said washed biofilm to detect and/or localize the biofilm.

As used herein, the term "labeled biofilm" means that at least one of the populations of substance portions comprised in a composition according to the first aspect of the present invention has diffused into the pores of the biofilm, thus labelling the biofilm with the respective dye.

According to a preferred embodiment, step a) comprises forming a bond between the composition of the first aspect of the invention, specifically the labeled substance portions and/or the cleaning substance portions comprised in said composition, and the biofilm, and/or forming an ionic bond between the composition and the biofilm, and/or forming a non-covalent intermolecular interaction between the composition and the biofilm. According to another preferred embodiment, step a) is performed by continuously flowing a liquid comprising said labeled component over a surface. Preferably, the reaction conditions are selected in accordance with Table 2.

According to another preferred embodiment, step b) is performed under influence of high shear forces, preferably by spraying off, even more preferably by spraying off with a high pressure cleaner. In this way, excess composition that is not bound to the biofilm may be removed thus reducing unspecific detection signals.

According to another preferred embodiment, step c) comprises detecting each of the dyes as defined herein comprised in each of the labeled substance portions. Said detection may be performed subsequently or simultaneously. The inventor of the present disclosure has surprisingly found that a biofilm can thus be readily detected, e.g. with the eye or using simple equipment such as a UV lamp. No specialized equipment, such as a microscope, or knowledge in microbiology is needed. Therefore, the method according to the second aspect of the present invention can be conveniently performed by any staff production. Additionally or alternatively, the following detection means can be used: spectrofluorometry, fluorescence microscopy, fluorescence polarization (FP), fluorescence lifetime spectroscopy (FLIM), total internal reflection fluorescence (TIRF), Flow cytometry, Förster resonance energy transfer (FRET), fluorescence correlation spectroscopy (FCS), fluorescence detection plate reader, optical coherence tomography with fluorescence (OCT-F), Raman fluorescence combination, and/or Hand-held fluorescence detectors. It is understood that the means of detections is chosen in accordance with the respective dye(s) to be detected.

According to another preferred embodiment, the method further comprises a step d) of characterizing the biofilm. For that, it is essential that the composition according to the first aspect of the present invention comprises characterizing substance portions (K_{Ch}) according to the fourth aspect of the present invention.

According to another preferred embodiment, the method further comprises a step e) of removing the biofilm. For that, the composition according to the first aspect of the present invention may comprise cleaning substance portions (K_{Cl}) according to the seventh aspect of the present invention. Upon activation of said cleaning substance portions (K_{Cl}), the cleaning agent is efficiently delivered to the biofilm which is thus removed from the surface.

Alternatively, if any one of the first labeled substance portions (K_{L}1), of the second labeled substance portions (K_{L}2), third labeled substance portions (K_{L}3), and/or further labeled substance portions (K_{L}n) further comprises a cleaning agent as defined herein step e) further comprises activation said labeled substance portions, preferably by at least one of a change in pressure, a change in pH, UV radiation, a change in osmotic pressure, a change in temperature, a change in light intensity, and/or by contact to a biofilm. Upon activation of said labeled substance portions, the cleaning agent is efficiently delivered to the biofilm which is thus removed from the surface.

According to a third aspect, the present invention relates to the use of a composition according to the first aspect of the present disclosure for detecting and/or localizing a biofilm, preferably to the use of a composition according to the first aspect of the present disclosure for detecting and/or localizing a biofilm on a surface. Said surface is not particularly limited.

For example, the surface may be a surface of a reaction tank. Such reaction tank is frequently used in Pharma industry, food industry, or the like. Another example of a surface is the surface of an implant, medical device, or dental implant. Detecting and optionally characterizing a biofilm on such implant, medical device, or dental implant is essential for patient safety and prevention of infections, in particular infections with antibiotic resistant pathogens.

The composition according to the first aspect of the present disclosure may also advantageously be used in laboratory settings to ensure sterility of laboratory benches and workplaces. In food packaging, it may ensure quality, safety, and shelf life of a food product. In water treatment, its use may increase water quality and reduce need for disinfectants.

The composition according to the first aspect of the present invention may also be advantageously used in oil and gas industry. In the oil and gas industry, biofilms can develop in wellbore systems, pipelines, and equipment components. They can reduce production efficiency, promote corrosion, and hinder cleaning and maintenance processes.

In conclusion, the composition according to the first aspect of the present disclosure may be used in all kinds of industries.

### Characterizing a biofilm

According to a fourth aspect, the present invention relates to a composition for characterizing a biofilm, preferably a composition for characterizing a biofilm on a surface.

According to the fifth aspect, the composition for characterizing a biofilm comprises a plurality of first characterizing substance portions (K_{Ch}1), wherein each of the first characterizing substance portions (K_{Ch}1) comprises a first dye as defined herein, and wherein each of the characterizing substance portions (K_{Ch}1) is provided with a first functional group (F_{Ch}1) as defined herein, and a first linker (L_{Ch}1) as defined herein.

According to a preferred embodiment of the present invention, the composition according to the fourth aspect further comprises a plurality of second characterizing substance portions (K_{Ch}2), wherein each of the second characterizing substance portions (K_{Ch}2) comprises a second dye as defined herein, and wherein each of the second characterizing substance portions (K_{Ch}2) is provided with a second functional group (F_{Ch}2) as defined herein, and a second linker (L_{Ch}2) as defined herein.

According to another preferred embodiment of the present invention, the composition according to the fourth aspect further comprises a plurality of third characterizing substance portions (K_{Ch}3), wherein each of the third characterizing substance portions (K_{Ch}3) comprises a third dye as defined herein, and wherein each of the third characterizing substance portions (K_{Ch}3) is provided with a third functional group (F_{Ch}3) as defined herein, and a third linker (L_{Ch}3) as defined herein.

According to another preferred embodiment of the present invention, the composition according to the fourth aspect further comprises at least one further plurality of characterizing substance portions (K_{Ch}n), wherein each of the at least one further pluralities of characterizing substance portions (K_{Ch}n) comprises a further dye as defined herein, and wherein each of the at least one further characterizing substance portions (K_{Ch}n) is provided with a further functional group (F_{Ch}n) as defined herein, and a third linker (L_{Ch}n) as defined herein.

According to a further preferred embodiment, the first characterizing substance portions (K_{Ch}1), and/or the second characterizing substance portions (K_{Ch}2), and/or the third characterizing substance portions (K_{Ch}3), and/or the at least one further characterizing substance portions (K_{Ch}n) is/are formed in accordance with Table 2.

It is understood that each of the functional groups (F_{Ch}) may independently be adapted to bind to a biofilm in a specific stage of biofilm evolution as defined herein. According to a preferred embodiment, each of the functional group (F_{Ch}) is independently selected according to Table 2.

The inventor of the present invention has surprisingly found that the different functional groups as defined herein, such as in Table 2, bind to different molecules of the EPS matrix of a biofilm. The composition of the biofilm can thus be determined by detecting the respective dye(s). For example, if an EPS matrix of a biofilm consists primarily of sugar and DNA, then, for it may be provided that the red labeled substance portions bind to sugar structures and the green labeled substance portions bind to DNA. This makes the biofilm look red/green. With a different composition, the biofilm is e.g. purple, yellow and orange, depending on the combination of functional group and respective dye.

The mean particle diameter of the first characterizing substance portions (K_{Ch}1), and/or the second characterizing substance portions (K_{Ch}2), and/or the third characterizing substance portions (K_{Ch}3), and/or the at least one further characterizing substance portions (K_{Ch}n) is not particularly limited but may preferably be in the range of from about 1 nm to 500 µm.

Providing a characterizing composition comprising multiple, such as two, three, four, or more populations of characterizing substance portions allows for characterizing the biofilm without the need of microbiological analysis methods. It is understood that the functional groups of the characterizing substance portions enable the binding to the biofilm. Upon detecting each of the dyes bound to the biofilm, conclusions about the composition of said biofilms can be dawn based on the detectable dyes.

According to another preferred embodiment, each of the dyes as defined herein, i.e. first, second, and third dye as defined herein, is independently a fluorescent dye or a non-fluorescent dye, with the provision that the first dye, the second dye (if present), and the third dye (if present) are different from each other. In other words, it is understood that each of the types of labeled substance portions comprised in a composition according to the first aspect of the present invention comprises a different dye as defined herein.

In a fifth aspect, the present invention relates to a method for characterizing a biofilm, preferably to a method for characterizing a biofilm on a surface.

According to a fifth aspect, the present invention relates to a method for characterizing a biofilm, preferably a method for characterizing a biofilm on a surface, the method comprising the steps of a) contacting a composition according to the fourth aspect of the present invention with a biofilm to provide a labeled biofilm; b) washing said labeled biofilm to provide a washed biofilm; and c) detecting the composition on said washed biofilm to characterize the biofilm.

It is understood that step a) comprises forming a bond between the characterizing substance portions comprised in the composition of the fourth aspect of the invention (specifically the characterizing substance portions) via the respective functional group.

According to another preferred embodiment, step a) is performed by continuously flowing a liquid comprising said labeled component over a surface. According to a further preferred embodiment, step a) is carried out under the conditions according to Table 2.

According to another preferred embodiment, step b) is performed under influence of high shear forces, preferably by spraying off, even more preferably by spraying off with a high pressure cleaner. In this way, excess composition that is not bound to the biofilm may be removed thus reducing unspecific detection signals.

According to another preferred embodiment, step b) may be performed by rinsing.

It is understood that step b) is carried out under conditions that remove excess composition. Labeled substance portions comprised in the composition that have diffused into the pores of a biofilm are not removed, but remain in the biofilm where they can be detected subsequently. Also, labeled substance portions that are bound to the biofilm by means of a functional group are not removed, but remain bound to the biofilm where they can be detected subsequently.

According to another preferred embodiment, step c) comprises detecting each of the dyes as defined herein comprised in each of the labeled substance portions. Said detection may be performed subsequently or simultaneously. The inventor of the present disclosure has surprisingly found that a biofilm can thus be readily characterized by a method of detection as defined herein, e.g. with the eye or using simple equipment such as a UV lamp. No specialized equipment, such as a microscope, or knowledge in microbiology is needed. Therefore, the method according to the second aspect of the present invention can be conveniently performed by any staff production. It is understood that each of the dyes is selected so that it can be detected independently, and its emission spectrum does not overly with a bio fluorescence of a biofilm.

According to another preferred embodiment, the method of the fifth aspect further comprises a step e) of removing the biofilm. For that, the composition according to the forth aspect of the present invention further comprises cleaning substance portions (K_{Cl}) according to the seventh aspect of the present invention. Upon activation of said cleaning substance portions (K_{Cl}), the cleaning agent is efficiently delivered to the biofilm which is thus removed from the surface.

According to a sixths aspect, the present invention relates to the use of a composition according to the fourth aspect of the present disclosure characterizing a biofilm, preferably to the use of a composition according to the fourth aspect of the present disclosure for characterizing a biofilm on a surface. Said surface is not particularly limited.

For example, the surface may be a surface of a reaction tank. Such reaction tank is frequently used in Pharma industry, food industry, or the like. Another example of a surface is the surface of an implant, medical device, or dental implant. Detecting and optionally characterizing a biofilm on such implant, medical device, or dental implant is essential for patient safety and prevention of infections, in particular infections with antibiotic resistant pathogens.

### Removing a biofilm

According to a seventh aspect, the present invention relates to a composition for removing a biofilm, preferably a composition for removing a biofilm from a surface. Accordingly, the composition for removing a biofilm comprises a plurality of cleaning substance portions (K_{Cl}), wherein each of the cleaning substance portions (K_{Cl}) comprises at least one cleaning agent, and wherein each of the cleaning substance portions (K_{Cl}) is provided with at least one fourth functional group (F_{Cl}4) as defined herein and at least one fourth linker (L_{Cl}4) as defined herein, wherein the at least one fourth linker (L_{Cl}4) is adapted to bind the at least one fourth functional group (F_{Cl}4) to the at least one cleaning substance portion (K_{Cl}).

According to a further preferred embodiment, the cleaning substance portions (K_{Cl}) is/are formed in accordance with Table 2.

According to another preferred embodiment, each of the cleaning substance portions (K_{Cl}) can be activated. According to a further preferred embodiment, each of the cleaning substance portions (K_{Cl}) can be activated by at least one of a change in pressure, a change in pH, UV radiation, a change in osmotic pressure, a change in temperature, a change in light intensity, and/or by contact to a biofilm.

According to a further preferred embodiment, each of the cleaning substance portions (K_{Cl}) comprises one, two, or three of the cleaning agents as defined herein. According to another preferred embodiment, each of the cleaning substance portions (K_{Cl}) comprises one cleaning agent as defined herein, and the composition comprises one, two, three, or more types of cleaning substance portions (K_{Cl}), wherein each type of cleaning substance portion (K_{Cl}) comprises a different cleaning agent.

According to another preferred embodiment, the cleaning substance portions (K_{Cl}) have a mean particle diameter in the range of from about ~1 nm to about 500 µm. According to another preferred embodiment, the cleaning substance portions (K_{Cl}) have a mean particle diameter in the range of from about 0.5 µm to about 100 µm, or in the range of from about ~1 nm to about 50 µm, or in the range of from about 0.5 µm to about 40 µm. It is understood that providing cleaning substance portions with the above particle diameters ensures that the cleaning agent can be delivered through the pores of the biofilm and hence efficiently removes the biofilm, e.g. removes the biofilm from a surface.

The inventor of the present disclosure has surprisingly found that the composition according to the third aspect of the present invention comprising a plurality of cleaning substance portions (K_{Cl}) is particularly effective in delivering a cleaning agent to the biofilm. Without wishing to be bound by theory, it is assumed that the cleaning substance portions may infiltrate into the pores of a biofilm and hence deliver the cleaning agent to the biofilm directly.

According to a further aspect of the present invention, the composition according to the seventh aspect of the present invention may be incorporated into a coating. In other words, the present invention is directed to a coating comprising a composition according to the third aspect of the present invention. Said coating may be applied to a surface and hence efficiently prevent the formation of a biofilm on said surface comprising the coating.

In an eights aspect, the present invention relates to a method of removing a biofilm, preferably to a method of removing a biofilm from a surface. Accordingly, the methods of the eighth aspect of the present invention comprises the steps of a) contacting a composition according to the first aspect or according to the fourth aspect, or according to the seventh aspect of the present invention with a biofilm; and b) activating the cleaning substance portions (K_{Cl}) to remove the biofilm. It is understood that step a) may be carried out in the same manner as defined in the second aspect of the present invention.

It is understood that step a) comprises forming a bond between the cleaning substance portions comprised in the composition of the seventh aspect of the invention via the respective fourth functional group (F_{Cl}4).

According to another preferred embodiment, step a) is performed by continuously flowing a liquid comprising said labeled component over a surface. According to a further preferred embodiment, step a) is carried out under the conditions according to Table 2.

Upon activation of said cleaning substance portions (K_{Cl}), the cleaning agent is efficiently delivered to the biofilm which is thus removed from the surface.

According to a ninth aspect, the present invention relates to the use of a composition according to the seventh aspect of the present disclosure for removing a biofilm, preferably to the use of a composition according to the seventh aspect of the present disclosure for removing a biofilm from a surface. Said surface is not particularly limited.

### Definitions and general embodiments

As used herein, the "mean particle diameter" refers to the arithmetic average particle diameter. As used herein, the mean particle diameter is determined by dynamic light scattering, preferably by dynamic light scattering on a Malvern Zeta-sizer instrument. Alternatively, the mean particle diameter is determined by a microscopy method comprising determining the diameter of at least 10 substance portions and calculating the mean particle diameter.

It is understood, that the mean particle size of a substance portion, such as a capsule, can be tuned by the stirring speed, ultrasonic amplitudes, ultrasonic duration, the concentration of shell material, and/or the curing time during manufacturing as shown in the table 1:

**Table 1 Conditions for adjusting the mean particle size of a substance portion, such as a capsule**

| mean substance portion diameter (µm) | Stirring speed (rpm) | Ultrasonic amplitude (%) | Ultrasound duration (minutes) | shell material concentration (%) | Curing time (hours) |
|---|---|---|---|---|---|
| 10 | 500-700 | 30-40 | 1 | 0,5 | 6 |
| 8 | 700-900 | 40-50 | 1,5 | 0,75 | 4 |
| 6 | 900-1.200 | 50-60 | 2 | 1 | 3 |
| 4 | 1,200-1,500 | 60-70 | 2,5 | 1,25 | 2,5 |
| 2 | 1,500-2,000 | 70-80 | 3 | 1,5 | 2 |

As used herein, the term "substance portion" can generally comprise (nano- and/or micro and/or matrix)-capsules, dots, as well as geometric or non-geometric shapes, stripes, spheres, particles, ellipses, lines, tracks, matrix, and the like. In this context, nanocapsules typically have a size in the nanometer range (i.e., size smaller than 1000 nm), while microcapsules can also have capsules in the size range of a few millimeters, such as up to 2 mm. The terms substance and substance portion are used synonymously in the present application.

In general, the substances are placed in the core (also referred to as nucleus) of the respective capsules and are surrounded by one or more shells. These substances can be in solid, liquid or gaseous form. Alternatively, the substance portions have a solid shell material and the dye is distributed throughout the solid shell material.

### Composition

According to another preferred embodiment, the composition according to the first aspect of the present invention, or the composition according to the fourth aspect of the present invention, or the composition according to the seventh aspect of the present invention is a powder composition or a slurry, or a dispersion.

Preferably, the composition according to the first aspect of the present invention, or the composition according to the fourth aspect of the present invention, or the composition according to the seventh aspect of the present invention is in the form of an aqueous dispersion. Further preferable, the aqueous dispersion comprises a catalyst adapted to catalyze a reaction between a functional group and a biofilm.

### Linker

As used herein, the term "linker" refers to a structure that is adapted to connect a respective functional group (such as a first, second, third, or fourth functional group) to a respective substance portion (such as a first, second, or third labeled substance portion, or a cleaning substance portion). As used herein, the linker is selected from the group consisting of selected from the group consisting of star polymers, biopolymers, alkanes (e.g., (C₁-C₂₀)alkanes), alkenes (e.g., (C₂-C₂₀)alkenes), alkynes (e.g., (C₂-C₂₀)alkynes), aliphatic chains, proteins, silk, polysaccarides, cellulose, starch, chitin, nucleic acid, synthetic polymers, homopolymers, polyethylenes, polypropylenes, polyvinyl chloride, polylactam, natural rubber, polyisoprene, copolymers, random copolymers, gradyent copolymer, alternating copolymer, block copolymer, graft copolymers, arcylnitrile butadyene styrene (ABS), styrene acrylonitrile (SAN), buthyl rubber, polymer blends, polymer alloy, inorganic polymers, polysiloxanes, polyphophazenes, polysilazanes, ceramics, basalt, isotactic polymers, syndiodactic polymers, atactic polymers, linear polymers, crosslinked polymers, elastomers, thermoplastic elastomers, thermosetting polymers, semi-crystalline linkers, thermoplastics, cis-trans polymers, conducting polymers, supramolecular polymers, linear polymers, polymers with multivalence, star-shaped polyethylene glycols, self-assembled monolayers (SAM), carbon nanotubes, ring-shaped polymers, dendrimers, ladder polymers and or similar substances, and supramolecular polymers.

According to another preferred embodiment, the linker may be a hydrophilic linker or a hydrophobic linker.

It is understood that a hydrophilic linker may render the surface of a respective substance portion hydrophilic which improves the diffusion of the respective substance portion into a biofilm having a hydrophilic surface or hydrophilic properties. It is understood that a hydrophobic linker may render the surface of a respective substance portion hydrophobic which improves the diffusion of the respective substance portion into a biofilm having a hydrophobic surface or hydrophobic properties.

### Substance portion and capsule shell

It is generally possible to produce substance portions, such as capsules, by physical methods, chemical methods, physiochemical methods and/or similar methods. It is generally possible for the shell of the substance portions, such as capsules, to comprise at least one polymer (preferably PVA or PMMA), wax resin, protein, lipids, maleic formaldehydes, resins, carbohydrates, proteins, polysaccharide, gum arabic, maltodextrin, inulin, metal, ceramic, acrylate, microgel, phase change material, and/or one or more other substances, as well as combinations thereof. It is generally possible for the substance portions to be formed with linear polymers, polymers with multivalence, star-shaped polyethylene glycols, self-assembled monolayers (SAM), carbon nanotubes, ring-shaped polymers, dendrimers, ladder polymers, and/or the like.

As used herein, the "shell material" may form a solid particle that comprises the respective dye (the dye is distributed throughout the shell material), or may form a shell around a core, wherein said core comprises the dye and/or a cleaning agent.

According to a preferred embodiment, a substance portion may be provided with a hydrophilic shell material. Thus, the respective substance portion may have a hydrophilic surface which improves the diffusion of the respective substance portion into a biofilm having a hydrophilic surface or hydrophilic properties. According to another preferred embodiment, a substance portion may be provided with a shell material comprising a positive charge. Thus, the respective substance portion may have a charged surface which improves the diffusion of the respective substance portion into a biofilm having a hydrophilic surface, negatively charged surface, or hydrophilic properties. According to another preferred embodiment, a substance portion may be provided with a shell material comprising a negative charge. Thus, the respective substance portion may have a charged surface which improves the diffusion of the respective substance portion into a biofilm having a hydrophilic surface, positively charged surface, or hydrophilic properties. According to another preferred embodiment, a substance portion may be provided with a hydrophobic shell material. Thus, the respective substance portion may have a hydrophobic surface which improves the diffusion of the respective substance portion into a biofilm having a hydrophobic surface or hydrophobic properties.

### Functional groups

According to another preferred embodiment, each of the labeled substance portions may be provided with at least one functional group and at least one linker wherein the linker is adapted to bind the at least one functional group to said labeled substance portion. In other words, it is preferable that each of the first labeled substance portions (K_{L}1) is provided with at least one first functional group (F_{L}1) and at least one first linker (L_{L}1), wherein the at least one first linker (L_{L}1) is adapted to bind the at least one first functional group (F_{L}1) to the at least one first labeled substance portion (K_{L}1). According to another preferred embodiment, each of the second labeled substance portions (K_{L}2) is provided with at least one second functional group (F_{L}2) and at least one second linker (L_{L}2), wherein the at least one second linker (L_{L}2) is adapted to bind the at least one second functional group (F_{L}2) to the at least one second labeled substance portion (K_{L}2). According to another preferred embodiment, each of the third labeled substance portions (K_{L}3) is provided with at least one third functional group (F_{L}3) and at least one third linker (L_{L}3), wherein the at least one third linker (L_{L}3) is adapted to bind the at least one third functional group (F_{L}3) to the at least one third labeled substance portion (K_{L}3). The same preferably applies to each of the further labeled substance portion populations, if present.

The inventor of the present disclosure has surprisingly found that providing a substance portion with at least one functional group may improve the characterization of the biofilm. As used herein, a functional group, such as the at least one first functional group (F_{L}1), and/or if present the at least one second functional group (F_{L}2), and/or if present the at least one third functional group (F_{L}3), and/or of present the at least one fourth functional group (F_{Cl}4) is adapted to bind to a biofilm. Preferably, a functional group as used herein is adapted to bind to the biofilm during one stage of development of said biofilm, more preferably wherein the functional group is adapted to bind to a biofilm during a stage of initial colonization of a surface by a flagellated cell; or during a stage of start of biofilm formation through cell adhesion; or during a stage of exponential growth; or during a stage of nutrient deficiency in the center of said biofilm; or during a stage of emigration through sporulation and flagellated cells.

As used herein, the first, second, third, fourth, and any further functional group may preferably be selected from the group consisting of -OH, -COOH, -NH₂, or groups adapted to chelate a metal ion, such as Ca²⁺, Mg²⁺, polyethylene glycole (PEG), polyethylene glycole (PEG), carbonyl group (-C=O), -SH, -PO43-, alkenyl (-C=C-), alkynyl group (-C=C-), ester (-COO-), amide (-CONH2), -NO2, -CN, -R-SH, imine (-C=NH), epoxide group, acyl group (-COR), anhydride (-CO-O-CO-), azo group (-N=N-), peroxide group (-O-O-), carbamate group (-NHCOO-), urethane group (-NHCOO-), guanidino group (-C(=NH)-NH₂), isocyanate group (-N=C=O), sulphonyl group (-SO₂-), sulphoxide group (-SO-), sulfonic acid group (-SO₃H), thiocyanate group (-SCN), diazo group (-N₂⁺), hydrazino group (-NH-NH₂), hydroperoxide group (-OOH), oxazolidinone group (heterocyclic ring with O, N and carbonyl), quaternary ammonium group (-NR₄⁺), epoxy group (three-membered ring with oxygen), isonitrile group (-NC), acetal group (-CH(OCH₃)₂), hemiacetal group (-CH(OH)(OR)), ketal group (-C(OR)₂), hemiketal group (-C(OH)(OR)), enol group (-C=C-OH), vinyl group (-CH=CH₂), allyl group (-CH₂-CH=CH₂), benzyl group (-CH₂-C₆H₅), indole group (heterocyclic structure with a benzene and pyrrole ring), furan group (heterocyclic ring with one oxygen atom), thiazole group (heterocyclic ring with sulphur and nitrogen), pyridyl group (-C₅H₄N, derivative of pyridine), pyrimidinyl group (heterocyclic ring with two nitrogen atoms), imidazole group (heterocyclic ring with two nitrogen atoms), thioether group (-R-S-R'), thionyl group (-SO), isothiocyanate group (-N=C=S), carbazole group (polycyclic aromatic system with nitrogen), boronic acid group (-B(OH)₂), and the like.

In that, it is understood that -OH and -COOH may bind to the polysaccharide matrix of a biofilm exhibiting -OH groups, and/or to protein rich components exhibiting -NH₂ groups. Metal ions or protonated amino groups may bind to an organic substances, such as minerals in the biofilm. Furthermore, ionic functional groups, -NH₂, and -OH functional groups may bind to acidic groups in the EPS matrix or to DNA or RNA-based structures in the biofilm. Lipophilic functional groups may bind to unpolar parts of the biofilm by hydrophobic interactions. Therefore, the binding between the labeled substance portions and the biofilm can be improved.

In other words, according to a preferred embodiment, each of the functional groups as disclosed herein is independently selected from the group consisting of -OH, -COOH, -NH₂, groups adapted to chelate a metal ion, such as Ca²⁺, Mg²⁺, and the like.

According to another preferred embodiment, each of the functional groups is independently adapted to bind to a component of the EPS matrix of a biofilm, such as a sugar, polysaccharide, DNA, RNA, lipid, a protein, an inorganic component, such as a mineral, or the like.

According to a preferred embodiment, a substance portion may be provided with a hydrophilic functional group. Thus, the respective substance portion may have a hydrophilic surface which improves the diffusion of the respective substance portion into a biofilm having a hydrophilic surface or hydrophilic properties. According to another preferred embodiment, a substance portion may be provided with a functional group comprising a positive charge. Thus, the respective substance portion may have a positively charged surface which improves the diffusion of the respective substance portion into a biofilm having a hydrophilic surface, negatively charged surface, or hydrophilic properties. According to another preferred embodiment, a substance portion may be provided with a functional group comprising a negative charge. Thus, the respective substance portion may have a negatively charged surface which improves the diffusion of the respective substance portion into a biofilm having a hydrophilic surface, positively charged surface, or hydrophilic properties. According to another preferred embodiment, a substance portion may be provided with a hydrophobic functional group. Thus, the respective substance portion may have a hydrophobic surface which improves the diffusion of the respective substance portion into a biofilm having a hydrophobic surface or hydrophobic properties.

According to another preferred embodiment, each functional group may independently be adapted to bind to a biofilm in a specific stage of development. Preferably, each functional group is adapted to bind to a specific target structure in a biofilm, which may preferably selected in accordance to a given stage of biofilm development, and is independently selected according to table 2:

**Table 2: Selection of functional group adapted to bind to a biofilm in a given stage of development**

| **Target structure in biofilm** | **functional group** | **preferred temperature (step a)** | **preferred contact duration (step a)** | **preferred cleaning agent** | **preferred shell material** |
|---|---|---|---|---|---|
| polysaccharid matrix | -OH or -COOH | 40 to 60 °C | 30 min to 60 min | alkaline cleaner (pH>9) | PVA |
| proteins | -OH- or -COOH | 20 to 40 °C | 1 h to 2 h | enzymatic | PLA |
| inorganic components, e.g. minerals | metal ion (e.g. Ca²⁺, Mg²⁺), or -NH₃⁺ | 40 to 50 °C | 2 h to 3 h | complexing (e.g. EDTA, citrate) | ethyl cellulose (EC) |
| EPS matrix (acidic groups) | -NH₂, -OH, metal ions (e.g. Ca²⁺, Mq²⁺) | 20 to 30 °C | 2 h to 3 h | acidic (e.g. citric acid, acetic acid) | PMMA |
| hydrophobic layers | -NH₂, -OH | 10 to 30 °C | 1 h to 2 h | detergent (e.g., SDS, LLS) | polycaprola ctone (PCL) |
| lipophilics / fats | lipid, nonpolar group | 20 to 40 °C | 6 h to 12 h | disinfectant (ethanol, isoprop.) | chitosan |
| DNA | metal ions (e.g. Ca²⁺, Mq²⁺) | 20 to 30 | 30 min to 60 min | enzymatic (e.g. DNAse) | HPMC |
| RNA | metal ions (e.g. Ca²⁺, Mq²⁺) | 20 to 30 °C | 30 min to 60 min | enzymatic (e.g. RNAse) | alginates |

### Surfaces

As used herein, a biofilm is preferably present on a surface. As used herein, a surface may be any surface that is susceptible to formation of a biofilm, such as the surface of a reactor, the surface of a working bench, the surface of a pipe, a filter, a surface of a medical device, a surface of an implant, a surface of a dental prosthetic, a food packaging, or the like.

### Cleaning agent

As used herein, the term "cleaning agent" refers to at least one cleaning agent selectd from the group consisting of at least one enzymatic cleaning agent, at least one oxidative agent, at least one antibiotic agent, at least one bacteriophage, at least one disinfectant, and at least one quorumsensing inhibitor.

According to a preferred embodiment, the cleaning agent is at least one enzymatic cleaning agent, preferably at least one enzymatic agent selected from the group consisting of at least one protease, at least one amylase, at least one cellulose, at least one lipase, at least one laccase, at least one lysozyme, at least one pectinase, at least one chitinase, and at least one peroxidase.

According to another preferred embodiment, the at least one cleaning agent is at least one oxidative agent, preferably at least one oxidative agent selected from the group consisting of hydrogen peroxide (H₂O₂), sodium hypochlorite, water, peracetic acid, chlorine dioxide, potassium permanganate, a persulfate, such as sodium persulfate, hypobromous acid, and bromine.

According to another preferred embodiment, the at least one cleaning agent is at least one antibiotic agent selected from the group consisting of ethanol, isopropanol, rifampin, vancomycin, gentamicin, ciprofloxacin, tobramycin, daptomycin, azithromycin, linezolid, colistin, and metronidazole.

According to another preferred embodiment, the at least one cleaning agent is at least one bacteriophage.

According to another preferred embodiment, the at least one cleaning agent is at least one quorumsensing inhibitor, preferably at least one quorumsensing inhibitor selected from the group of a furanone, C-30 Furanone, ajoene, baicalin, gallium (III) nitrate, naringenin, a brominated furanone, RNAIII-inhibiting peptide, 5-fluorouracil, vanillin, hamamelitannin, ibuprofen, and curcumin.

### Dye

As used herein, each of the dyes, e.g. first, second, and third dye, is independently a fluorescent dye or a non-fluorescent dye.

According to a preferred embodiment, a fluorescent dye is selected from the group consisting of fluorescein, rhodamine, texas red, Cy3, Cy5, 4',6-diamidino-2-phenylindole, fluorescein isothiocyanate, Alexa Fluor 488, Alexa Fluor 647, tetramethylrhodamine isothiocyanate, green fluorescent protein (GFP), mCherry, yellow fluorescent protein (YFP), a quantum dot, preferably Qdot 525 or Qdot 655, and boron-dipyrromethene (BODIPY).

According to a preferred embodiment, a non-fluorescent dye is selected from the group consisting of methyl orange, bromocresol green, malachite green, sudan stain, preferably Sudan I, Sudan II, Sudan III, Sudan IV, Sudan Black B, or Sudan Red, an azo dye, hematoxylin, eosin, 3,3'-diamonobenzidine, coomassie brilliant blue, neural red, methylene blue, and bromophenol blue.

According to another preferred embodiment, the dye is selected from the group consisting of carotenoids (e.g. beta-carotene, lycopene), chlorophyll (green plant pigment), curcumin (yellow pigment from turmeric), anthocyanins (e.g. in berries, red to blue), indigo (blue dye from plants), carmine/cochineal (red dye from scale insects), saffron (yellow dye from the saffron flower), synthetic dyes (such as, methyl orange (indicator dye), malachite green (green dye), crystal violet (violet dye), rhodamine B (bright pink), fluorescein (yellow-green fluorescent), eosin (pink staining), brilliant blue FCF (blue), tartrazine (yellow), allura red AC (red)) azo dyes (such as methyl red, congo red, sudan I-IV (lipid dyes, yellow to red), amaranth (red)), phthalocyanines, copper phthalocyanine (blue), chlorinated copper phthalocyanine (green), fluorescent dyes, FITC (fluorescein isothiocyanate), texas red, DAPI (blue), 4. Hoechst dye (blue fluorescent), food colorings (such as E100 (curcumin), E101 (riboflavin), E120 (carmine), E140 (chlorophyll), E160a (beta-carotene), E162 (betanin, beet), E171 (titanium dioxide, white pigment)), textile dyes (such as reactive red 120, direct blue 1, acid black 194, vat orange 1, disperse yellow 3), and other industrial dyes (such as alizarin (red), indanthrene blue, nigrosine (black), and aniline dyes).

These dyes are particularly easy to detect after binding of the labeled substance portions to a biofilm, e.g. with the eye or by using simple equipment, such as a UV lamp. Thus, no specialized equipment or extensive knowledge in microbiology is needed.

### Example

To produce 100 g of labeled substance portions with a homogeneous size distribution, a hydrophobic dye (Sudan Red) is encapsulated in an oil-in-water emulsion with PLGA as the shell material. First, 2 g of Sudan Red is dissolved in 20 g vegetable oil and 15 g PLGA is dissolved in 50 mL dichloromethane with stirring. The dye-oil solution is emulsified into the PLGA solution (ultrasound: 50 % amplitude, 2 minutes).

The primary emulsion is added to 500 ml of 1 % PVA solution and homogenized at 1,000 rpm, followed by renewed ultrasonication to refine the size distribution. Curing is carried out by solvent evaporation at room temperature (4 hours, 500 rpm).

After centrifugation (4,000 rpm, 10 minutes) and triple washing with water, the microsubstance portions are dried at 40 °C. The final product (approx. 100 g) has a mean particle diameter of 5 µm and an encapsulation efficiency of over 90 %.

## Claims

1. A composition for detecting and localizing a biofilm (preferably a biofilm on a surface), the composition comprising a plurality of first labeled substance portions (K_{L}1), wherein
a) each of the first labeled substance portions (K_{L}1) comprises a first dye; and wherein
b) the first labeled substance portions (K_{L}1) have a mean particle diameter in the range of from about 1 nm to about 40 µm.

2. The composition according to claim 1, wherein each of the first labeled substance portions (K_{L}1) is provided with at least one first functional group (F_{L}1) and at least one first linker (L_{L}1), wherein the at least one first linker (L_{L}1) is adapted to bind the at least one first functional group (F_{L}1) to the at least one first labeled substance portion (K_{L}1).

3. The composition according to claim 1 or 2, wherein the composition further comprises a plurality of second labeled substance portions (K_{L}2), wherein
a) each of the second labeled substance portions (K_{L}2) comprises a second dye; and wherein
b) the second labeled substance portions (K_{L}2) have a mean particle diameter in the range of from about 3 µm to about 8 µm, and the first labeled substance portions (K_{L}1) have a mean particle diameter in the range of from about 1 µm to about 4 µm;
preferably wherein each of the second labeled substance portions (K_{L}2) is provided with at least one second functional group (F_{L}2) and at least one second linker (L_{L}2), wherein the at least one second linker (L_{L}2) is adapted to bind the at least one second functional group (F_{L}2) to the at least one second labeled substance portion (K_{L}2).

4. The composition according to any one of claims 1 to 3, wherein the composition further comprises a plurality of third labeled substance portions (K_{L}3), wherein
a) each of the third labeled substance portions (K_{L}3) comprises a third dye; and wherein
b) the third labeled substance portions (K_{L}3) have a mean particle diameter in the range of from about 6 µm to about 10 µm, and the first labeled substance portions (K_{L}1) have a mean particle diameter in the range of from about 1 µm to about 4 µm;
preferably wherein each of the third labeled substance portions (K_{L}3) is provided with at least one third functional group and at least one third linker, wherein the at least one third linker is adapted to bind the at least one third functional group to the at least one third labeled substance portion (K_{L}3).

5. The composition according to any one of claims 1 to 4, wherein each of the first dye, second dye, and third dye is independently a fluorescent dye or a non-fluorescent dye, with the provisio that the first dye, the second dye, and the third dye are different from each other.

6. The composition according to claim 5, wherein the fluorescent dye is selected from the group consisting of fluorescein, rhodamine, texas red, Cy3, Cy5, 4',6-diamidino-2-phenylindole, fluorescein isothiocyanate, Alexa Fluor 488, Alexa Fluor 647, tetramethylrhodamine isothiocyanate, green fluorescent protein (GFP), mCherry, yellow fluorescent protein (YFP), a quantum dot, preferably Qdot 525 or Qdot 655, and boron-dipyrromethene (BODIPY).

7. The composition according to claim 5, wherein the non-fluorescent dye is selected from the group consisting of methyl orange, bromocresol green, malachite green, sudan stain, preferably Sudan I, Sudan II, Sudan III, Sudan IV, Sudan Black B, or Sudan Red, an azo dye, hematoxylin, eosin, 3,3'-diamonobenzidine, coomassie brilliant blue, neural red, methylene blue, and bromophenol blue.

8. The composition according to any one of claims 1 to 7, wherein the composition further comprises a plurality of cleaning substance portions (K_{Cl}), wherein each of the cleaning substance portions (K_{Cl}) comprises at least one cleaning agent;
preferably wherein each of the cleaning substance portions (K_{Cl}) is provided with at least one fourth functional group (F_{Cl}4) and at least one fourth linker (L_{Cl}4), wherein the at least one fourth linker (L_{Cl}4) is adapted to bind the at least one fourth functional group (F_{Cl}4) to the at least one cleaning substance portion (K_{Cl}).

9. The composition according to claim 8, wherein the cleaning substance portions (K_{Cl}) can be activated, wherein the cleaning substance portions (K_{Cl}) can be activated by at least one of a change in pressure, a change in pH, UV radiation, a change in osmotic pressure, a change in temperature, a change in light intensity, and/or by contact to a biofilm.

10. The composition according to any one of claims 8 or 9, wherein the at least one cleaning agent is
a) at least one enzymatic cleaning agent, preferably at least one enzymatic agent selected from the group consisting of at least one protease, at least one amylase, at least one cellulose, at least one lipase, at least one laccase, at least one lysozyme, at least one pectinase, at least one chitinase, and at least one peroxidase; and/or
b) at least one oxidative agent, preferably at least one oxidative agent selected from the group consisting of hydrogen peroxide (H₂O₂), sodium hypochlorite, peracetic acid, chlorine dioxide, potassium permanganate, a persulfate, such as sodium persulfate, hypobromous acid, and bromine; and/or
c) at least one antibiotic agent, preferably at least one antibiotic agent selected from the group consisting of ethanol, isopropanol, rifampin, vancomycin, gentamicin, ciprofloxacin, tobramycin, daptomycin, azithromycin, linezolid, colistin, and metronidazole; and/or
d) at least one bacteriophage; and/or
e) at least one quorumsensing inhibitor, preferably at least one quorumsensing inhibitor selected from the group of a furanone, C-30 Furanone, ajoene, baicalin, gallium (III) nitrate, naringenin, a brominated furanone, RNAIII-inhibiting peptide, 5-fluorouracil, vanillin, hamamelitannin, ibuprofen, and curcumin.

11. The composition according to any one of claims 8 to 10, wherein the cleaning substance portions (K_{Cl}) have a mean particle diameter in the range of from about 0.5 µm to about 20 µm.

12. A method for detecting a biofilm, the method comprising the steps of
a) Contacting a composition according to any one of claims 1 to 11 with a biofilm to provide a labeled biofilm;
b) Washing said labeled biofilm to provide a washed biofilm; and
c) detecting the composition on said washed biofilm to detect the biofilm.

13. The method according to claim 12, wherein step a) comprises forming a bond between the composition and the biofilm, and/or forming an ionic bond between the composition and the biofilm, and/or forming a non-covalent intermolecular interaction between the composition and the biofilm.

14. Use of a composition according to any one of claims 1 to 12 for detecting a biofilm.

15. Use of a composition according to any one of claims 8 to 12 for removing a biofilm.
